# EUROPEAN PATENT APPLICATION

(11) **EP 3 498 699 A1**
(43) Date of publication of application: **19.06.2019**
(21) Application number: 17207110.2
(22) Date of filing: 13.12.2017
(51) Int. Cl.: C07D 307/46

(54) **CHROMATOGRAPHY METHOD FOR THE PURIFICATION OF FURFURAL DERIVATIVES**

(71) Applicant: BASF SE, 67056 Ludwigshafen am Rhein (DE)
(72) Inventor: NEU, Volker, 67056 Ludwigshafen (DE); LAMOTTE, Stefan, 67056 Ludwigshafen (DE); DUEFERT, Svenia, 67056 Ludwigshafen (DE); KINDLER, Alois, 67056 Ludwigshafen (DE); ZIER, Melanie, 67056 Ludwigshafen (DE); SA GOMES, Pedro, 67056 Ludwigshafen (DE)
(74) Representative: BASF IP Association

(57) **Abstract**

A liquid chromatography process for the purification of furfural derivative(s), comprising:
a. Introducing a feed mixture comprising at least one furfural derivative and other compounds onto a chromatographic bed comprising a silicate-based material as stationary phase, and
b. Flushing a liquid comprising an organic acid as mobile phase through the chromatographic bed producing an eluate comprising at least one fraction enriched in at least one furfural derivative and at least one fraction enriched in the other compounds.

## Description

The present invention relates to a liquid chromatography method for the purification of furfural derivative(s), comprising:
a. Introducing a feed mixture comprising at least one furfural derivative and other compounds onto a chromatographic bed comprising a silicate-based material as stationary phase, and
b. Eluting a liquid comprising an organic acid as mobile phase through the chromatographic bed producing an eluate comprising at least one fraction enriched in at least one furfural derivative and at least one fraction enriched in the other compounds.

Furfural derivatives, which are obtained from renewable raw materials and can be converted easily by chemical reactions to compounds which can be used industrially are increasingly of importance for chemical syntheses. Furfural derivatives are for example 5-(Hydroxymethyl)furfural, 5-(Acetoxymethyl)furfural, 5-(Methoxymethyl)-furfural, 5-(Chloromethyl)furfural and Furfural.

For example, 5-hydroxymethylfurfural (HMF) or derivatives are well-known and can be produced from hexoses by various methods (WO2014/199306, WO2016/207025, WO2014/033289). 2,5-furandicarboxylic acid is readily obtainable from HMF (WO2017/076947, WO2015/197699, WO2016/028488, WO2010/132740) and is suitable as dicarboxylic acids for producing polymers, such as polyesters or polyurethanes, and can replace other dicarboxylic acids from non-renewable raw materials in industrial applications.

HMF is generally produced by acid-catalyzed dehydration of hexoses such as glucose or fructose. Other furfural derivatives like 5-(Acetoxymethyl)furfural (WO2017/076956), 5-(Methoxymethyl)furfural, 5-(Chloromethyl)furfural are obtained by this reaction in the presence of acetic acid (M. Bicker et al., Journal of Supercritical Fluids (2005), 36(2), 118-126; WO2016/168233) or alkyl acetates (WO2017/076942), methanol (WO2012/091570) or HCI (WO2014/066746, WO2014/062303), respectively. Furfural is a side product of such reactions.

The reaction products obtained are solutions which, besides the HMF, comprise unreacted starting materials (e.g. sugars), solvent(s) and/or by-products like levulinic acid a levulinic esters, formic acid and/or humins.

Humins are a class of organic oligomeric and/or polymeric compounds obtained by dehydration of mono-, oligo- and polysaccharides, in particular pentoses and/or hexoses, e.g. fructose and/or glucose. They are insoluble in pure water at a wide pH-range. The humins arise by additional condensation of sugars with furfural and hydroxymethylfurfural, which are products of the dehydration of mono-, oligo- and polysaccharides. The structure of humins is mainly irregular, but structural motifs such as ethers, acetals, esters and aromatic components occur.

During the synthesis of furfural derivatives from sugars sometimes only a partial conversion of the starting materials is performed in order to avoid the formation of by-products. In general, the solutions obtained therefore comprise unreacted starting materials such as hexoses or oligomers or polymers composed of hexoses. In the case of higher conversions, the amount of by-products like humins increases.

Separating off the furfural derivatives from the reaction solution which comprise starting materials or by-products like humins of the HMF synthesis is complex and hinders the accessibility of furfural derivatives. Further, the separation of furfural derivatives from other polar components such as solvents can be extremely challenging due to thermal instability of the mentioned furfurals and their tendency to react with humins resulting in humins of even higher molecular weight.

For example, Feroz Kabir Kazi et al. describe in Chem Eng. J. 169 (2011), pages 329-338 separating off the HMF from the acidic reaction solution by a complex extraction method using an organic solvent (butanol); a solution of HMF in butanol is obtained.

DE-A 3601281 discloses a reaction of fructose to HMF in water with a subsequent chromatographic separation method in which firstly any organic solvents are removed and the aqueous HMF solution is separated using an ion exchange column. The HMF fraction obtained is crystallized. The yield of HMF is low. Separation from polar aprotic solvents which could provide a higher yield is not possible by this purification method.

A further complex method of separating off HMF from the reaction solution is the conversion of the HMF to another compound which is easier to separate off, optionally followed by a back-conversion to HMF after separation has taken place. For example, according to Mark Mascal and Edward B. Nikitin in 2008 Angew. Chemie vol. 47, pages 7924-7926, HMF is converted to the more stable 5-(Chloromethyl)furfural and then converted again to HMF or derivatives thereof.

D.W. Brown et al. describe in J. Chem. Tech. Biotechnol., 1982, Vol. 32, pages 920-924, attempts to completely remove a solvent by distillation lead to rapid carbonization of the target product HMF.

Furfural derivatives should be present for further syntheses in the purest possible form. Of particular suitability for further syntheses like oxidations are solutions of furfural derivatives in an organic acid such as acetic acid or aqueous solutions which at best do not comprise by-products or residual starting materials, or comprises them in very small amounts. Methods known hitherto for producing furfural derivatives or aqueous solutions thereof with adequate purity are extremely complex and expensive.

It was a primary object of the invention to provide a method with which furfural derivative(s) can be purified in a manner as simple and effective as possible, furfural derivative(s) are obtained at the same time in the purest form possible and furfural derivative(s) are therefore separated off directly as completely as possible from unreacted starting materials, solvents or by-products of the synthesis.

This object is achieved by a liquid chromatography for the purification of furfural derivative(s), comprising:
a. Introducing a feed mixture comprising at least one furfural derivative and other compounds onto a chromatographic bed comprising a silicate-based material as stationary phase, and
b. Eluting a liquid comprising organic acid as mobile phase through the chromatographic bed producing an eluate comprising at least one fraction enriched in at least one furfural derivative and at least one fraction enriched in the other components.

The application of chromatography using a silicate-based material as stationary phase and organic acid as mobile phase for the purification of the furfural derivative(s) is superior to thermal separation processes due to the low temperatures generally used in chromatographic processes which prevent thermally induced decomposition of the target furfural derivative(s). Using this technique, HMF and other furfural derivative(s) can be obtained as solution in an organic acid without significant losses of product as observed by thermal separation processes. Further, the purified stream of the furfural derivative(s) can directly be submitted to e.g. a further oxidation step to furandicarboxylic acid without the necessity to completely remove the solvent.

The organic solvent may be selected from the group consisting of acetic acid, propionic acid and formic acid and mixtures thereof. The organic solvent is preferably acetic acid.

Using silica-based adsorbents in combination with basic compounds like NMP may lead to partial dispersion or solvation of the adsorbent material. It is also known, that strong adsorption or tailing effects can occur in such cases. Surprisingly, the combination of silica-based material (adsorbents) and an organic acid, preferably acetic acid, as mobile phase in liquid chromatography for the separation of furfural derivative(s) like HMF or AcMF from basic solvents (pKa>7) such as amides does not show that effect. Compared to other adsorbents like acidic polymer resins, silicate-based material in combination with an organic acid give a good separation of furfural derivative(s), e.g. HMF and basic compounds (e.g. N-methylpyrrolidone) whereas other tested resins lack a significant separation efficiency. Surprisingly, a quality of the furfural derivative(s) in organic acid, in particular acetic acid, can be obtained, that is suitable for direct oxidation to furandicarboxylic acid.

The separation is aimed at the separation and purification of the furfural derivative(s) as target.

The feed mixture comprises the compounds to be separated by the chromatography, optionally in the presence of the organic acid acting as a solvent.

Preferably, the feed mixture comprises at least one furfural derivative and other compounds and optionally organic acid, preferably acetic acid.

Preferably, the concentration of furfural derivative(s) in the feed mixture is 5 to 40 wt-% furfural derivative(s) based on the total weight of the feed mixture. More preferably the concentration of furfural derivative(s) in the feed mixture is 10 to 25 wt-% furfural derivative(s) based on the total weight of the feed mixture.

Preferably, the concentration of polar aprotic solvent in the feed mixture is 5 to 50 wt-% based on the total weight of the feed mixture. More preferably, the concentration of polar aprotic solvent in the feed mixture is 10 to 30 wt-% based on the total weight of the feed mixture.

Preferably, the concentration of said organic acid in the feed mixture is 20 to 75 wt-% organic acid based on the total weight the feed mixture.

Preferably, the feed mixture comprises:
5 to 40 wt-% , more preferably 10 to 25 wt-%, furfural derivatives,
5 to 50 wt-%, 10 to 30 wt-% polar aprotic solvent, and
20 to 75 wt-% organic acid
based on the total weight of the feed mixture,
wherein the sum does not exceed 100 wt-%.

The furfural derivative(s) may selected from the group consisting of 5-(Hydroxymethyl)furfural, 5-(Acetoxymethyl)furfural, 5-Methoxy(methyl)furfural, 5-(Chloromethyl)furfural, Furfural and mixtures thereof.

The mobile phase flows through the stationary phase and carries the components of the feed mixture with it. The feed mixture is not part of the mobile phase.

Different components require different times to pass through the stationary phase. The mobile phase comprises at least 90, 91, 92, 92, 94, 95, 96, 97, 98, 99, 99.5, 99.6, 99.7, 99.8, 99.9 or wt- % organic acid based on the total weight of the mobile phase.

The organic acid maybe selected from the group consisting of acetic acid, propionic acid and formic acid and mixtures thereof. Preferably, the organic acid is acetic acid. Acetic acid, propionic acid and formic acid shall be used in a concentrated form of at least 96 wt-%, at least 97 wt-%, at least 98 wt-%, wt-% at least 99 wt-%, at least 99,9wt- % based on the total weight of the organic acid.

Other compounds are e.g. polar aprotic solvents and/or humins, optionally resulting from the production process of the furfural derivative.

Humins are a class of organic oligomeric and/or polymeric compounds obtained by dehydration of mono-, oligo- and polysaccharides, in particular pentoses and/or hexoses, e.g. fructose and/or glucose. They are insoluble in pure water at a wide pH-range. The humins arise by additional condensation of sugars with furfural and hydroxymethylfurfural, which are products of the dehydration of mono-, oligo- and polysaccharides. The structure of humins is mainly irregular, but structural motifs such as ethers, acetals, esters and aromatic components occur.

Preferably, the polar aprotic solvents have a pH above 7 at 25°C. The polar aprotic solvents may be N-Methylpyrrolidone (NMP), N-Ethylpyrrolidon, Dimethylpropylenurea or mixtures thereof. Preferred is N-Methylpyrrolidone. Suitable silica-based material may be selected from the group consisting of bare silica gel, diol-bonded-silica gel,alkylated silica gel and mixtures thereof. Bare silica gel means silica gel with unmodified OH-groups. Bare silica gel means preferably that at least 95%, at least 96%, at least 97%, at least 98%, at least 99% or 100% of the free OH-groups are hydrolyzed. Diol-bonded-silica-gel means bare silica gel is reacted with (3-Glycidoxypropyl)Trimethoxysilane (CAS No: 2530-83-8) and subsequently the ether-ring is opened with an acid. Alkylated silica gel means with silical gel, wherein the OH-groups are reacted with organosilanes carrying an alkyl chain length of C2, C4, C8 or mixtures thereof. In a preferred embodiment, the silica-based material is bare silica gel.

The silica-based material may have a defined medium pore size of 25 to 100 Å, more preferably 25 to 60 Å. The medium pore size is measured by the BET adsorption method (Ralph K. Iler: The chemistry of silica. 1979, Wiley).

The silica-based material may have a mean particle size distribution in the range of 10 µm to 1000 µm, more preferably in the range from 100 µm to 750 µm, and more preferably in range of 150 to 500 µm and most preferably in the range of 200 to 250 µm.

The silica-based material may have a specific surface areaof 50 to 700 m²/g, preferably 100-500 m²/g. The specific surface area of silica-based material is measured via nitrogen adsorption. In this measurement the adsorbent surface is covered with nitrogen under different pressure and the partial pressure of the nitrogen is measured for the corresponding pressure which is applied to the sample. From this the pore size and pore volume can be determined. The derivation of the adsorption and desorption curve gives the pore size distribution of the adsorbent. By applying the Wheeler equation, the specific surface area can be calculated. Said method is described in detail by Ralph K. Iler: The chemistry of silica. 1979, Wiley.

The pressure levels for the chromatography process can vary over a wide range of from 0.05 MPa to 15 MPa (0.01 MPa to 15 MPa pressure drop across unit).

Typically, the temperature of the column(s) is limited from a lower level where the formation of crystals or particulates may be observed up to vaporization of the mobile phase. In one embodiment, the chromatography process is carried out at a temperature of 17 to 80°C. According to a preferred embodiment the process is carried out at constant room temperature from 17 to 40°C.

In one embodiment of the liquid chromatography process different temperature zones are used along the stationary phase.

In one embodiment of the liquid chromatography process temperature gradient is installed by injection of mobile phase.

In one embodiment of the liquid chromatography process the composition of the liquid mobile phase is varied continuously or step-wise during the elution.

In one embodiment of the liquid chromatography process the feed mixture is subjected to sequential multi-column chromatography separation by introducing the feed mixture into an apparatus comprising a plurality of chromatographic columns sequentially linked together, each column comprising a stationary phase, wherein the plurality of columns is connected in series and a valve arrangement comprising multiple inlets and outlets.

In one embodiment the liquid chromatography process comprises a sequential multi-column chromatography process in moving bed, wherein the method employs a plurality of columns connected in series and a valve arrangement comprising multiple inlets and outlets.

The moving bed can be simulated or an actual moving bed. Preferably the moving bed is a simulated moving bed. Bed means the stationary phase comprising the silica-based material.

Simulated moving bed chromatography means that valves of the interconnected columns are sequentially switched in the direction of the fluid flow. This means that the inlets (mobile phase, eluent) and outlets (eluate) are regularly changed. The time between switching of the valves is called a cycle. At the end of the cycle, each inlet and outlet is moved one step in the direction of the mobile phase. Therefore, a countercurrent movement of the stationary phase and the mobile phase is simulated by sequentially changing the in- and outlets. Eluate means the mobile phase further comprising the compounds to be separated and is obtained from the outlets. Subsequently the Eluate may be used as Eluent. Eluent means the mobile phase further comprising the compounds to be separated and may be introduced via the inlets.

The method of the sequential multi-column chromatography in a simulated or actual moving bed apparatus and the respective equipment are described in the prior art such as US 2,985,589, US 3,696,107, US 3,706,812 or "Sa Gomes and Rodrigues (2012) Chemical Engineering & Technology Special Issue: Preparative Chromatography and Downstream Processing Volume 35, 17-34 ".

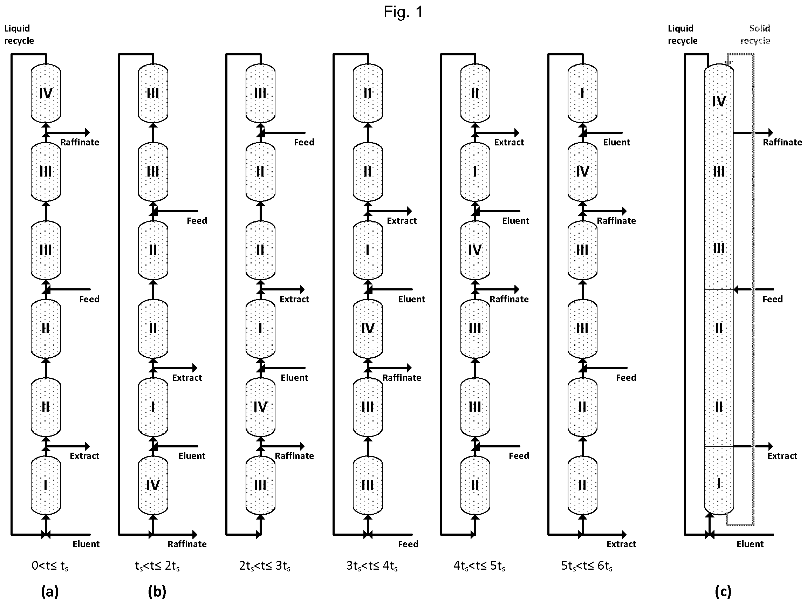

In a preferred embodiment the apparatus comprises a sequence of fixed bed columns, each column being equipped with inlets and outlet, in which the stationary phase is at rest in relation to a fixed referential, but where a relative movement between the fluid mobile phase and the stationary phase is caused by switching the inlet and outlet fluid streams to and from the columns from time to time (in the direction of the fluid flow). Thus, a kind of counter-current movement is created relatively to the fluid, this technology is called "Simulated Moving Bed" (SMB). A typical scheme is depicted in Figure 1.

By defining a *"section"* as the part of the SMB unit where the fluid flow rate is approximately constant (Figure 1), it is possible to find four different sections with different roles, by considering A - target molecule less retained in the column, and, B target molecule more retained in the column:
Section *I*: Regeneration of the stationary phase (adsorbent) (desorption of *B*, and *A* if still present, from the solid);
Section *II*: Desorption of *A* and adsorption of *B* (so that the extract, rich in *B*, is not contaminated with *A*);
Section *III:* Adsorption of *B* and desorption of *A* (so that the raffinate, rich in *A*, is not contaminated with *B*);
Section *IV*: Regeneration of the mobile phase -/eluent (adsorption of *A*, and *B* if still present, from the fluid).

The benefits of and similarity of related non-conventional SMB operating modes, such as Improved-SMB, Sequential-SMB, Varicol®, Powerfeed, Modicon, MCSGP, Outlet Swing Stream-OSS, JO or pseudo SMB, among others, is known and can be derived from classical SMB results by a skilled expert, as detailed for example in "Sa Gomes and Rodrigues (2012) Chemical Engineering & Technology Special Issue: Preparative Chromatography and Downstream Processing Volume 35, 17-34".

The respective equipment for carrying out the chromatography is commercially available and can be adapted by the skilled expert to the specific needs of the separation process, operated under different pumps, valves and configuration and columns in static position or as actual moving bed (AMB, CSEP, ISEP apparatus) as described in US 7,141,172.

For the purpose of the invention the following terms may be used:
An eluent portion enriched in a target molecule less retained in the column is also referred to as "raffinate".

An eluent portion depleted of a target molecule more retained in the column is also referred to as "extract".

The target molecule may either be the furfural derivative to be purified or a specific impurity to be removed. Thus, depending on the specific process scheme, in connection with the claimed invention the first eluent portion enriched in the furfural derivative may be either a raffinate or an extract as is explained in detail further below.

Step (A) provides for a feed mixture comprising the furfural derivative and other compounds, optionally dissolved in a solvent. The upper limit is depending on the solubility of the given product in the solvent. The feed mixture composition may be different from the eluent stream fed to the chromatographic unit, to allow solvent gradient operation.

Step (B) comprises subjecting the feed mixture to a chromatographic separation by introducing the feed mixture into an SMB apparatus comprising a plurality of chromatographic columns sequentially linked together, each column comprising a stationary phase.

The number of columns used in each apparatus is not particularly limited. A skilled person would easily be able to determine an appropriate number of columns depending the amount of material to be purified. The number of columns is typically 2 or more, preferably 4 or more, for example 4, 5, 6, 7, 8, 9 or 10 columns. In a preferred embodiment 5 or 6 columns, more preferably 6 columns. In another preferred embodiment the number of columns is 7 or 8 columns, more preferably 8 columns. Typically, there are no more than 25 columns, preferably no more than 20 columns, more preferably no more than 15 columns. In a particularly preferred embodiment the number of columns is 2 columns per section, a section being a part of the unit where the flow rate is approximately constant and defined by inlets and outlets. The inlets and/or outlets may be valves.

The dimensions of the columns are not particularly limited and will depend to some extent on the volume of the feed mixture to be purified, the stationary phase particle size and flow rates chosen for the specific separation process.

A skilled person will be able to determine appropriately sized columns. The inner diameter ("ID") of each column is typically between 0.1 m to 10 m, preferably between 1 m to 6 m. The length of each column is typically between 0.05 m to 2 m, preferably between 0.5 m to 1.5 m.

The flow rates to the columns are limited by maximum pressure drop across the series of columns and will depend on the column dimensions and particle size of the stationary phase. A person skilled in the art will be able to establish the required flow rate for each column dimension to ensure an efficient separation process. Larger diameter columns will typically require higher flow rates to maintain linear flow through the columns (Cf. Sa Gomes and Rodrigues (2012) Chemical Engineering & Technology Special Issue: Preparative Chromatography and Downstream Processing, Volume 35, 17-34). The stationary phase comprises a silicate-based material.
Low pressure units maybe operated with particles sizes from 50 µm and up to 1000 µm, preferably 100 to 500 µm, particularly preferred 200 -350 µm.

According to a particularly preferred embodiment the stationary phase is pre-treated by eluting with the mobile phase until stable retention times are reached.

According to one embodiment of the invention the first eluent portion and the second eluent portion recovered after a separation cycle can be independently of each other subjected to a concentration step.

The concentration step of the first eluent portion enriched in the furfural derivative and/or the second eluent portion depleted of the furfural derivative can be carried out by evaporation, drying or distillation.

According to another embodiment the concentration step of the first eluent portion enriched in the furfural derivative and the second eluent portion depleted of the furfural derivative is carried out by liquid extraction, membranes, crystallization, adsorption or other solvent recovery techniques.

The different eluent portions can be independently of each other treated by different methods, meaning that different methods may be used for the different eluent streams.

The chromatography process optionally includes pre-filtering of the feed mixture and/or mobile phase and/or eluate streams.

This pre-filtering can be carried out using adsorption filter beds, adsorption columns, flash chromatography or batch adsorption by stirring the mobile phase and/or eluate and/or feed mixture together with an adsorptive material followed by filtration. The filter beds can comprise silica-based materials, aluminas, molecular sieves, activated carbons, polymeric adsorbents, ion exchangers or other adsorbents. Preferably the pre-filtering comprises silica-based material as defined above alone or in combination with ion exchangers.

The pre-filtering step can be used either to remove humins or salts in the feed mixture, that may damage or alter the SMB separation behaviour by strongly adsorbing in the stationary phase, or other side impurities that accumulate in the mobile phase cycle, or even waste material prevenient from a defective mobile phase recovery procedure.

According to one embodiment the inventive process comprises a first filter step prior to a separation chromatography cycle by passing the feed mixture through a fixed filter bed of silica-based material or aluminas or molecular sieves or activated carbons or polymeric adsorbents such as cation or anion exchange resins for the removal of salts or catalyst traces or mixtures of thereof, positioned between the feed vessels and the chromatography apparatus.

According to another embodiment the inventive process comprises a second filter step after a separation chromatography cycle by passing the depleted eluent through a filter bed of silica-based material or aluminas or molecular sieves or activated carbons or polymeric adsorbents such as cation or anion exchange resins positioned in the eluent recycling zone.

According to a preferred embodiment the filter beds are pretreated with a solvent prior to the filtration step. Preferably the solvent is also used as the mobile phase. The filter bed can be pre-treated in more than one step, for instance by a first pre-treatment with water, followed by second pre-treatment with the solvent of the mobile phase.

According to another embodiment the inventive process comprises the step of subjecting the first eluent portion rich in the furfural derivative to a second simulated or actual moving bed separation process cycle. Depending on the sequence of purification steps the first eluent enriched in the furfural derivative can be either the raffinate or the extract.

According to yet another embodiment the process comprises one or more eluent concentration steps between the first and the second process cycle.

A preferred embodiment represents a process for the purification of furfural derivative(s), comprising using sequential multi-column size chromatography apparatus operated as a counter current moving bed wherein a process cycle comprises the steps of (A) providing a feed mixture comprising the furfural derivative and other compounds dissolved in organic acid in a feed vessel, (B) subjecting the feed mixture to a chromatographic separation by introducing the feed mixture into an apparatus comprising a plurality of chromatographic columns sequentially linked together, each column comprising a bed, (C) Eluting a liquid comprising an organic acid as mobile phase through the counter current moving bed, (D) after separation collecting a first eluent portion enriched in the purified furfural derivative and a second eluent portion depleted of the purified furfural derivative, (E) collecting the purified furfural derivative from the first eluent portion, and (F) recovery of the depleted eluent and recycling the depleted eluent from the mobile phase recovery zone into the process.

Optionally, the concentration of the furfural derivative in the raffinate obtained by the liquid chromatography method is higher than 1% w/w, preferably higher than 2% w/w, more preferably higher than 3% w/w. The raffinate may be further concentrated after the liquid chromatography.

In one embodiment the eluate comprising the furfural derivative obtained by the liquid chromatography is oxidized to produce 2,5-furandicarboxylic acid, preferably the eluate is not further purified before the oxidation.

In one embodiment the eluate comprising the furfural derivative obtained by the liquid chromatography is reduced to produce 2,5-bis(hydroxymethyl)furan and/or 2,5-bis(hydroxymethyl)tetrahydrofuran, preferably the eluate is not further purified before the oxidation.

The invention is further illustrated by the following figures:
Figure 1 depicts a 6 columns SMB unit with column arrangement nj=[1 2 2 1] over a complete cycle (from 0 to 6 tₛ, where tₛ is the ports switching time); (a) until the first switch; (b) from the first switch to the second; extract and raffinate the streams where the more and the less retained key compounds are collected, respectively.

By defining a "section" as the part of the SMB unit where the mobile phase flow rate is approximately constant (Figure 1), by considering A - target molecule less retained in the chromatography column, and B target molecule more retained in the chromatography column, it is possible to find four different sections with different roles:
Section I: Regeneration of the stationary phase (desorption of *B*, and *A* if still present, from the solid);
Section II: Desorption of A and adsorption of *B* (so that the extract, rich in *B*, is not contaminated with *A*);
Section III: Adsorption of *B* and desorption of *A* (so that the raffinate, rich in *A*, is not contaminated with *B*);
Section IV: Regeneration of the mobile phase (adsorption of *A*, and *B* if still present, from the fluid).

If one considers that at certain moment in the operation of an SMB unit the positions of the inlet and outlet ports are represented by Figure 1a, after a period of time (switching time, *tₛ*), all the inlets and outlets move one column in the direction of the mobile phase flow reaching Figure 1b. The same procedure will continue synchronously after each switching time until the initial location of all the streams is reencountered. When this happens, one cycle has been completed.

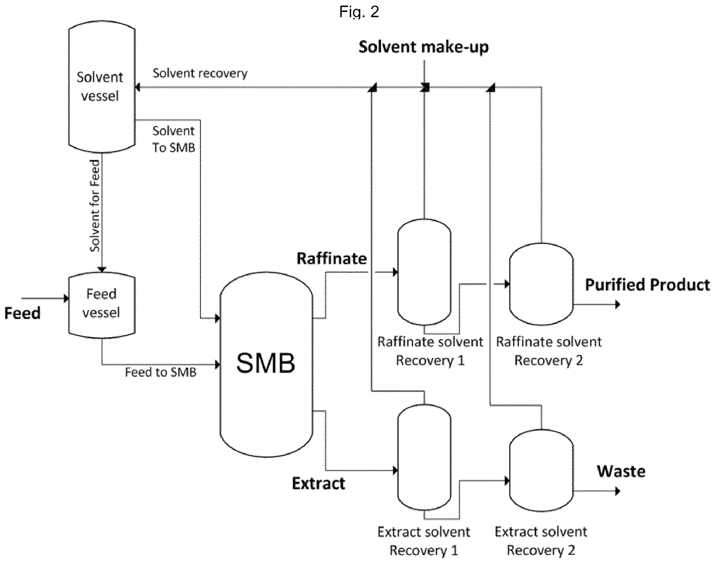

Figure 2 depicts the set-up of a one-step SMB unit for the purification of hydroxymethylfurfural with solvent recovery wherein the purified target product is collected from the raffinate stream.

Feed Mixture 1; Feed Vessel 2; Feed to SMB Unit 3; SMB Unit 4;
Extract Stream 5; Extract Solvent Recovery 1 6a; Extract Solvent Recovery 2 6b; Waste Stream 7; Raffinate Stream 8; Raffinate Solvent Recovery 1 9a; Raffinate Solvent Recovery 2 9b; Solvent Recovery Stream 9c; Purified Product Stream 10; Solvent Vessel 11; Solvent Stream to Feed Vessel 12a; Solvent Stream to SMB 12b; Solvent Make-up A . Solvent may also mean mobile phase.

Figure 3 shows the general setup of the HPLC-System applied in Example 1.

Figure 4 shows the elution profile of Example 1 showing an excellent separation of HMF and NMP.

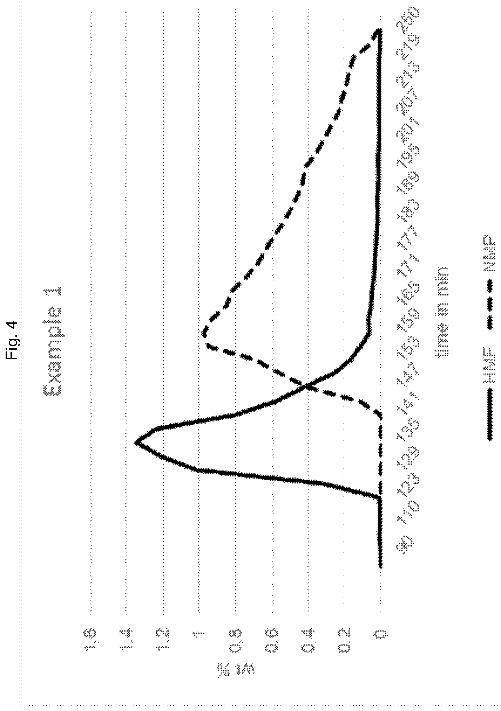

Figure 4 shows the elution profile of Example 2 (Comparative Example) showing no separation of HMF and NMP.

### Examples:

### Example 1:

To design a continuously operated SMB, pulse tests were generally performed using the following equipment:
HPLC system: Agilent 1100 Series including pump, degasser, a six-port switching valve and one DAD detector as well as one RI detector.

Additional HPLC Compact Pump (Bischoff) connected to the six-port valve of the HPLC system.

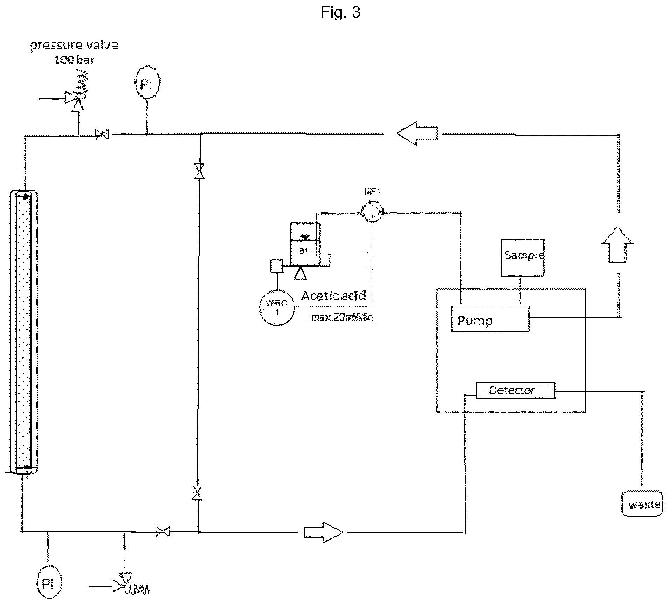

Column: Götec borosilicate column Super Compact 600-26 (600 mm long, 26 mm diameter) with a total volume of 338 mL. The general setup is shown in Figure 3:
The column was charged with 170.4 g of dry silica gel (pore size of 30 Å, particle size of 25-40µm) and flushed with 5 L of acetic acid (>99% purity) at a flow rate of 2.3 mL/min. Subsequently, the flow of acetic acid through the column was set to a rate of 2.6 mL/min. Injection of 5.74 g of the sample containing HMF, N-Methylpyrrolidone (NMP) and other components in acetic acid (13 wt% HMF, 21 wt% NMP based on the total amount of the feed (injection)) was performed at a flow rate of 4 mL/min within 86 seconds.

The experiment was run at a temperature of 296 K. Samples were collected continuously and regularly and they were analyzed for the products HMF and NMP using HPLC resulting in an elution profile as shown in Figure 4. Individual samples, which were analyzed for HMF and NMP by HPLC, were measured using the following method and equipment: Column ACQUILITY UPLC HSS T3 (1,8 µm 3,0x75 mm) by Waters, operated at 40°C, injection volume of 2 microliters, at a flow rate of 1mL/min, detection of HMF using UV-detection at a wavelength of 267 nm, detection of NMP using a RI-detector. Solvent: water/acetonitrile in different ratios (gradient) with 1wt% H3PO4. High purity samples of HMF(>98%) and NMP (>99%) were used for calibration.

The elution profile shows an excellent separation of HMF and NMP.

### Example 2 (Comparative example):

Column: Götec borosilicate column Super Compact 600-26 (600 mm long, 26 mm diameter) with a total volume of 338 mL. The general setup is shown in Figure 3:
The column was charged with 100 mL of water. Then 187.4 g of ion-exchange resin Amberlite XAD 761 (e.g. from Dow) was filled into the column and flushed with 3.6 L of water within 4 h. Subsequently the resin was flushed with acetic acid (>99% purity) at a flow rate of 10 mL/h for 1 h. Another 24 g of Amberlite XAD 761 were added to the column and flushed with 5.4 kg of acetic acid (>99% purity). Subsequently, the flow of acetic acid through the column was set to a rate of 5.3 mL/min. Injection of 1.0 g of the sample containing HMF, N-Methylpyrrolidone (NMP) and other components in acetic acid (ca. 5 wt-% HMF, 24 wt-% NMP based on the total amount of the feed (injection), sample contains minor amounts of humins, furfural, levulinic acid and other side components) was performed at a flow rate of 1.4 mL/min within 42.8 seconds. Detection of HMF was performed via the DAD-detector at a wavelength of 310 nm, whereas NMP detection was performed using the RI-detector.

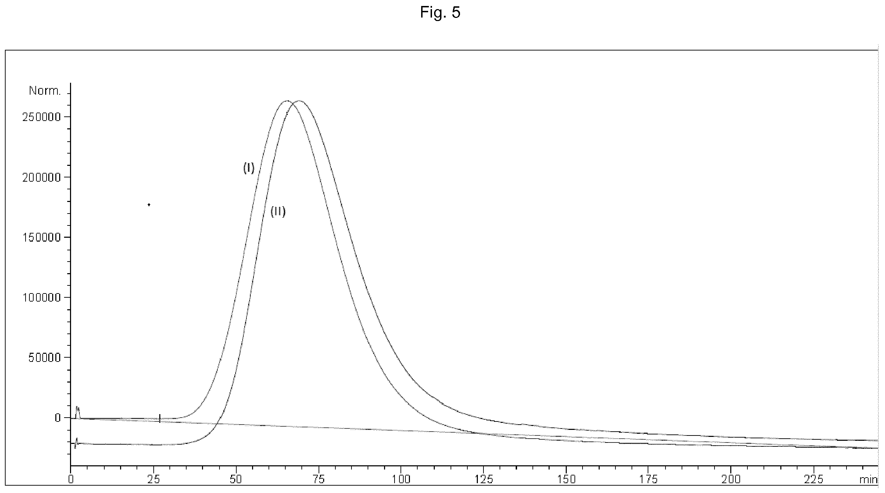

The separation of HMF (I) and NMP (II) was very inefficient even at very low amounts of HMF and NMP injected (cf. Figure 5).

## Claims

1. A liquid chromatography process for the purification of furfural derivatives, comprising:
a. Introducing a feed mixture comprising at least one furfural derivative and other compounds onto a chromatographic bed comprising a silicate-based material as stationary phase, and
b. Eluting a liquid comprising an organic acid as mobile phase through the chromatographic bed producing an eluate comprising at least one fraction enriched in at least one furfural derivative and at least one fraction enriched in the other compounds.

2. Process according claim 1, wherein the furfural derivative is selected from the group consisting 5-(Hydroxymethyl)furfural, 5-(Acetoxy-methyl)furfural, 5-Methoxy(methyl)furfural, 5-(Chloromethyl)furfural, Furfural and mixtures thereof.

3. Process according to anyone of the preceding claims, wherein the organic acid is selected from the group consisting of acetic acid, propionic acid, formic acid and mixtures thereof.

4. Process according to anyone of the preceding claims, wherein the organic acid is acetic acid.

5. Process according to anyone of the preceding claims, wherein the mobile phase comprises of at least 90 wt-% organic acid based on the total weight of the mobile phase.

6. Process according to anyone of the preceding claims, wherein the other compounds comprise polar aprotic solvents and/or humins.

7. Process according to claim 6, wherein the polar aprotic solvents are selected from the group consisting of N-Methylpyrrolidone, N-Ethylpyrrolidon, N,N'-Dimethylpropyleneurea and mixtures thereof.

8. Process according to anyone of the preceding claims, wherein the silica-based material is selected from the group consisting of bare silica gel, diol-bonded-silica gel, alkylated silica gel with an alkyl chain length of C2, C4 and/or C8 and mixtures thereof.

9. Process according claim 8, wherein the silica-based material has a defined medium pore size of 25 to 100 Å.

10. Process according to claims 8 or 9, wherein the silica-based material has a mean particle size distribution of 10 µm to 1000 µm.

11. Process according to anyone of claims 8 to 10, wherein the silica-based material has a specific surface area of 50 to 700 m²/g.

12. Process according to anyone of the preceding claims, wherein the chromatographic separation is carried out at 17°C to 80°C.

13. Process according anyone of the preceding claims, wherein different temperature zones are used along the stationary phase.

14. Process according claim 13, wherein a temperature gradient is installed by injection of mobile phase.

15. Process according to anyone of the preceding claims, wherein the composition of the liquid mobile phase is varied continuously or step-wise during the elution.

16. Process according to anyone of the preceding claims, wherein the concentration of furfural derivatives in the feed mixture is 5 to 40 wt-% furfural derivatives based on the total weight of the feed mixture.

17. Process according to anyone of the preceding claims, wherein the concentration of polar aprotic solvents in the feed mixture is 5 to 50 wt-% based on the total weight of the feed mixture.

18. Process according to anyone of the preceding claims, wherein the concentration of the furfural derivatives in the raffinate obtained by the liquid chromatography process is higher than 1wt-%, preferably higher than 3% wt% based on the total weight of the raffinate.

19. Process according to anyone of the preceding claims, wherein the feed mixture is subjected to sequential multi-column chromatography separation by introducing the feed mixture into an apparatus comprising a plurality of chromatographic columns sequentially linked together, each column comprising a stationary phase, wherein the plurality of columns is connected in series and a valve arrangement comprising multiple inlets and outlets.

20. Process according to claim 19, comprising using sequential multi-column size chromatography apparatus operated as a counter current moving bed wherein a process cycle comprises the steps of (A) providing a feed mixture comprising the furfural derivatives and other compounds dissolved in organic acid in a feed vessel, (B) subjecting the feed mixture to a chromatographic separation by introducing the feed mixture into an apparatus comprising a plurality of chromatographic columns sequentially linked together, each column comprising a stationary phase,
(C) Flushing a liquid comprising an organic acid as mobile phase through the counter current moving bed
(D) after separation collecting a first eluent portion enriched in the purified furfural derivatives and a second eluent portion depleted of the purified furfural derivatives,
(E) collecting the purified furfural derivatives from the first eluent portion, and (F) recovery of the depleted eluent and recycling the depleted eluent from the mobile phase recovery zone into the process.

21. Process according to anyone of the preceding claims, comprising a first filter step prior to the liquid chromatography by passing the feed mixture through a filter bed of silica-based material, aluminas, molecular sieves, activated carbon, polymeric adsorbents, ion exchanger or mixtures thereof.

22. Process according to anyone of the preceding claims, wherein the eluate is oxidized to produce 2,5-furandicarboxylic acid.

23. Process according anyone of the preceding claims, wherein the eluate is reduced to produce 2,5-bis(hydroxymethyl)furan and/or 2,5-bis(hydroxymethyl)tetrahydrofuran.
